# EUROPEAN PATENT SPECIFICATION

(11) **EP 4 201 381 B1**
(45) Date of publication and mention of the grant of the patent: **22.04.2026**
(21) Application number: 22383002.7
(22) Date of filing: 19.10.2022
(51) Int. Cl.: A61F 7/02, A61F 7/00, A61F 7/10

(54) **CRYOGENIC GARMENT**
KRYOGENISCHES KLEIDUNGSSTÜCK
VÊTEMENT CRYOGÉNIQUE

(30) Priority: 21.12.2021 ES 202132502 U
(43) Date of publication of application: 28.06.2023
(73) Proprietor: THALIA CAPITAL, S.L., 08302 Mataro (Barcelona) (ES)
(72) Inventor: Leon Cid, Joaquin, 08301 Mataro (Barcelona) (ES)
(74) Representative: Durán-Corretjer, S.L.P.

(56) References cited:
- EP-A1- 3 922 224
- US-A- 5 065 758
- US-A- 5 843 145
- US-A1- 2015 335 472

## Description

The present invention relates to a garment used to apply cold, for use in recovery following sporting activity.

The application of any agent to reduce the temperature of body tissues for therapeutic purposes is known as cryotherapy.

Cold can be applied locally using ice packs, instant ice packs, cold sprays, etc., or generally using ice baths, cryosaunas and the like.

The application of cold to body tissues and consequent temperature reduction thereof has a series of effects on said tissues, specifically:
- Analgesic effect. Cold reduces sensitivity, having a local sedative effect.
- Vasoconstriction. Cold causes blood vessels to contract, thereby reducing the blood supply to the area.
- Anti-inflammatory. When applied correctly, cold helps to reduce inflammation in muscles, tendons and ligaments caused by an injury or excessive physical activity.
- Muscle relaxation. Cold reduces spasticity.

The use of cryotherapy in sports is known on account of the extensive preventative and curative benefits thereof. In terms of prevention, applying cold to body tissues helps with muscle recovery and reduces fatigue, among other benefits. In terms of healing, applying cold helps to reduce the inflammation related to a large number of injuries, such as periostitis, tendinitis or bursitis etc., among other benefits.

Despite the benefits offered by cryotherapy in sporting activity, it is not in widespread use in part because it is difficult to apply. With the exception of elite sportspeople, few sportspeople have access to ice baths, cold-water baths, cryosaunas or the like. This means that most sportspeople who use cryotherapy for preventive or curative purposes use ice packs or cold packs, which are bags or packs that contain a chemical substance with a high heat capacity and a high latent heat of fusion. The nature of such packs, in particular the size thereof, means that cold can only be applied to a relatively specific area of the body. For example, multiple ice packs or cold packs are required to treat a relatively large area, such as the legs. The use of multiple packs brings related problems, in particular relating to attaching the packs to a specific part of the body. In addition to the foregoing, such use of multiple packs causes issues related to the uniformity of the cold applied.

Cooling recovery garments are known in the prior art. Spanish utility model ES 1147037 U discloses cooling recovery leggings that have in their interior one or more bag compartments containing a viscose gel able to maintain low temperatures without solidifying. Said recovery leggings also have an inner lining designed to prevent ice burns. One drawback of this embodiment is that all of the disclosed compartment designs make the garment very expensive to produce. In one embodiment in which the gel cannot be removed, the main drawback is having to create a gel pack with an anatomical shape. This with the subsequent incorporation of the pack into the leggings is a complex process, which results in a high retail price for said leggings, thereby considerably reducing the probability of commercial success. Furthermore, this embodiment would also suffer from a heavy weight, resulting in a relative loss of comfort. In an embodiment in which the gel can be removed to be cooled in a freezer or the like for subsequent reinsertion into the leggings to be used by the user, said leggings have a plurality of pockets designed to receive a plurality of gel bars. Each pocket has a respective Velcro^{®} closure. One of the drawbacks of this type of leggings is that removing and inserting the gel bars in the respective pockets can be somewhat awkward. Furthermore, this type of embodiment also has the problem of requiring the use of multiple gel bars of different lengths to achieve a good anatomical fit, which increases the range of gel bars required to mass produce this type of leggings, thereby increasing the sale price thereof and reducing the probability of commercial success.

Spanish utility model ES 1225460 U by the same applicant as the present invention discloses a cryotherapy garment that has several elongate horizontal receptacles designed to receive a cold-store material, in which each of said receptacles is formed by a closed fabric fold in the garment and each of said receptacles has two end openings for inserting a cylindrical pack of cold-store material. Said closed folds form receptacles suitable for receiving the pack of material during the same manufacturing process of the garment, and to be closed longitudinally by two closing lines using weaving techniques with circular sewing machines. The presence of two openings facilitates the simple insertion of packs of cold-store material. One opening enables insertion of a pack, while the other opening enables tools such as a cross-action clamp to be passed therethrough to insert the packs of cold-store material. The packs of cold-store material are concatenated capsules of malleable plastic, which obviates the need for a large stock of gel bars of different sizes, since a given number of capsules can be cut away to obtain a strip of cold-store material of suitable length for the destination receptacle, with the cut being made at a joining zone between capsules. The type of garment in document ES 1225460 U is a compressive garment made from an elastomeric material, which combines the effects of pressure and cold, and enables the packs to be inserted and held on account of the elasticity of the material. Despite resolving some of the problems, such as being easier to manufacture, not using Velcro^{®} closures, and the greater logistical versatility and adaptability provided by the horizontal concatenated capsules, which also enhance mobility, there are still some drawbacks. The garment has the advantage of being more versatile and adaptable to the shape of the body. However, the garment has some drawbacks: the cylindrical shape of the capsules results in a suboptimal contact surface with the body, and the plastic gaps used to cut to different lengths results in a loss of space of at least 1 cm between adjacent capsules where there is no cold-store material. Furthermore, said concatenated capsules are difficult to insert on account of the elongate narrow shape of the receptacles, and tools or double openings may be required to insert said capsules depending on the circumstances. Moreover and similarly to other packs of cold-store material for cryogenic garments known in the prior art, these capsules have the drawback of being malleable, which further increases the variability of the fit with the body, and consequently the application of cold to the target areas. These packs tend to be somewhat fragile, and misuse or simple wear caused by friction from use can damage the pack and cause leaks of cryogel. Moreover, cryogel is not an easily obtainable material, making it more expensive and difficult to replace.

One objective of the present invention is to provide a product that facilitates the use of cryotherapy, in particular in the sporting domain, thereby resolving the aforementioned problems in the existing prior art. For this purpose, the present invention discloses a cryotherapy garment including various receptacles designed to receive a pack of cold-store material, in which each of said receptacles is formed by a closed fabric fold in the garment, each of said receptacles having an opening for insertion of the pack of cold-store material, and each receptacle receiving a respective pack, characterized in that said packs are made of a semi-rigid and have a shape as defined in independent claim 1. Preferred embodiments are disclosed in the dependent claims

Semi-rigid material means a material that can retain its shape when idle or when subjected to slight forces or stresses, but that can deform elastically and automatically return to its original shape if said stresses exceed certain values, and that can deform non-elastically or break if said stresses exceed even greater values.

The cold-store material is preferably provided in individual semi-rigid plastic packs. This prevents said packs from changing shape in response to the temperature of the cold-store material, for example when said material passes into its liquid form, thereby retaining a specific shape especially designed to enhance the efficiency of the transmission of cold to the body of the user.

Making the individual packs from semi-rigid material also has the advantage of enabling the shape of the packs to be designed specifically to find an optimum balance between the greatest possible cryogenic effect and ease of insertion of the packs into the receptacles.

In the present document, cold-store material preferably refers to any material with a high heat capacity and/or a high latent heat of fusion. The cold-store material is usually and preferably characterized by having a high thermal inertia and in particular a low melting point. The type of cold-store material most commonly found in the prior art is a eutectic material or gel. Other options are possible and can provide logistical, commercial or adaptability advantages for different products. Any type of cold-store material can be used for the present invention.

In one particularly advantageous embodiment of the invention, the cold-store material is distilled water. In another equally advantageous embodiment the material is alcohol water. Both embodiments are less costly than cryogenic gels, and logistically simpler to obtain and therefore to replace, as well as being adaptable to any shape of pack, which in this case are preferably small and rigid, on account of which low viscosity may be important to ensure that the packs are filled to maximum capacity.

Preferably, each pack of cold-store material has at least one flat face. Said flat face is designed to face the user's body, being in contact, following insertion thereof into an opening of a receptacle, with the surface of the garment designed to come into contact in turn with the user of the garment. More preferably, said flat face is the face with the largest surface area of the at least one pack. Even more preferably, the at least one pack of cold-store material has a pointed end to facilitate insertion of said at least one pack into the respective receptacle thereof. The face with the largest surface area is the face designed to face the body, thereby maximizing the potential heat conduction capacity.

Preferably, said at least one flat face has the same imprint as a conventional clothes iron. Preferably, said at least one flat face is formed by three straight sides, with two right angles therebetween, and two curved sides that form a point. One straight side is preferably a flat rear end, forming two right angles with the lateral sides, that start straight and curve tangentially at a given point eventually converging into a pointed end, said at least one flat face being symmetrical about its longitudinal axis. More preferably, the pack extends perpendicularly upwards from said flat face with the greatest surface area on the three sides joined by right angles, and extends upwards at an acute angle at the pointed end, thereby creating a three-dimensional point. Said three-dimensional point facilitates the initial insertion of the pack into a receptacle, without substantially reducing the contact surface with the user. Furthermore, the fact that the bullets have right angles except at the pointed end minimizes the space between said bullets, which are arranged next to one another, which also enhances the effect of the cryogenic treatment.

Even more preferably, considering said flat face with the larger surface area in the design of the pack to be the lower face, the pack has an upper flat face that is parallel to the lower flat face at the end that is not pointed, and that also curves tangentially downwards at the same point at which the lateral sides start to curve, joining the lateral sides to form the aforementioned three-dimensional point with a curved top surface. Even more preferably, the edges formed between all of the different faces are rounded to obviate sharp corners or edges that could scratch or wear the fabric, and in turn further facilitate insertion of the packs into the receptacles. By way of illustrative example, the bullets can preferably have an aerodynamic shape like a high-speed or bullet train.

In a preferred embodiment, the approximate dimensions of the bullets are as follows: 10 mm maximum height, 17 mm maximum width and 40 mm long, of which 20 mm has straight sides and the other 20 mm has curved sides, edge radii of 2 mm, lateral side radii of 30.17 mm, and radius of the top surface of the point of 32 mm. Other dimensions are also possible and the invention should not be considered to be limited to these dimensions.

The present invention enables the production of compressive garments that combine pressure with cold. This combines the benefits of cryotherapy with the benefits of using garments providing differential and uniform pressure. For this purpose, the fabric of the garment preferably includes an elastomer.

The presence of an opening per closed fold in combination with the use of a fabric with elastic properties provides enhanced holding of the packs in the receptacle. The openings of the receptacles are smaller in dimension than the packs, since the garment is made of an elastic material that enables the openings to open large enough to enable the packs to enter the receptacles, and the garment then adapts elastically to fit the shape of the pack, fastening the pack by elastic compression inside the receptacle.

In some embodiments, the longitudinal ends and one opposite end of the opening of said receptacle can be joined or interwoven together. In some embodiments, each of the longitudinal ends and one opposite end of the opening of said receptacle can be interwoven or joined to the garment. Preferably, said fold or folds are closed on both longitudinal sides and one of the ends thereof, thus forming the receptacles.

When the garment is being worn and the user of the garment is in a standing position, the receptacles preferably form small vertical pockets. Preferably, the opening is found at the upper end of the receptacle. Advantageously, said closure is made using weaving techniques. This enables the receptacles to be produced simultaneously with the garment, for example using a circular knitting machine.

Said vertical pockets are preferably grouped adjacent to one another along the longest sides thereof, forming horizontal rows of two or more such vertical pockets. The horizontal rows preferably extend about the entire perimeter of the limb or body to be covered. More preferably, the garment has one or more of said horizontal rows arranged above one another such that the vertical pocket receptacles can form a grid or mesh pattern.

Preferably, the production of the garment and the arrangement of the receptacles ensure that the individual packs are spaced apart regularly. The gaps between the pockets or receptacles that make up the horizontal rows are as small as possible to ensure that the receptacles are as close as possible, thereby optimizing the cold application effect. As mentioned previously, the distance between said horizontal rows can preferably be the same as the distance between adjacent pockets, thereby creating the grid or mesh pattern, but alternatively, depending on the desired pattern, the gap between different receptacles containing the respective cold-store material can be variable, i.e. the gap between the different receptacles with the respective cold-store material varies within the garment. This variation in the gaps between the different receptacles can be used for aesthetic or functional reasons, i.e. depending on the desired effect, the part of the body being treated, etc., or a combination of the two.

Grouping the vertical pockets into horizontal rows both facilitates the mobility of said limbs or parts of the body and insertion of the bullets, since the more pronounced curvature of one limb or part of the body occurs incrementally as the individual bullets are positioned about the perimeter of the limb or part of the body, with the transverse dimension of the bullet being the smallest dimension and therefore the closest to a continuous curve.

The fact of providing the cold-store material in small individual packs also enhances adaptability, since the smaller elements provide greater resolution, enabling tighter fits to more complex shapes.

In another embodiment of the invention, the pockets can be arranged in the horizontal rows according to a quincunx pattern, offset against contiguous rows. This arrangement of the pockets can further facilitate insertion of the packs, since the presence of another pack in another of the rows does not interfere with insertion of a new pack, since the openings between packs are between the packs in contiguous rows.

Although the materials mentioned above are preferably used to make cryotherapy garments according to the present invention, said garments can also be made using other materials or fabrics.

In a particularly advantageous embodiment, the cryotherapy garment can be leggings, i.e. a garment designed to cover the lower limbs of the user. Said leggings can be long, usually reaching approximately as far as the ankle, or short, reaching to above the knee of the user. The receptacles preferably cover the area from the iliac crest to the ankles, with the exception of the zones corresponding to the inside of the legs, genital area and intergluteal cleft.

In another embodiment, the garment can cover the user's torso, arranging receptacles in the torso area. Accordingly, for example, the cryotherapy garment according to the present invention can be a shirt, i.e. a garment designed to cover the torso. Said shirt can have straps, i.e. no sleeves, short sleeves or long sleeves.

In an alternative embodiment, the cryotherapy garment is a sleeve, i.e. a garment designed to cover only the user's arms, fully or partially.

In another alternative embodiment, the cryotherapy garment is a call sleeve, i.e. a garment designed to cover the user's calf.

In another alternative embodiment, the cryotherapy garment is a set of garments comprising any combination of the following garments: long leggings, short leggings, shirt, sleeve, and calf sleeve.

The above list of garment types should not be understood to be exhaustive and the garment according to the present invention can, inter alia, comprise combinations thereof, for example a jumpsuit.

Drawings showing an embodiment of a cryotherapy garment according to the present invention are attached to enhance comprehension by way of explanatory but non-limiting example.
Figure 1 is a front elevation view of an example embodiment of a set of cryotherapy garments according to the present invention.
Figure 2 is a rear elevation view of an example embodiment of a cryotherapy garment according to the present invention.
Figure 3 shows a detail of the structure that forms the receptacles arranged in a grid or matrix pattern.
Figure 4 is the reverse of Figure 3, showing the inner face of the receptacles.
Figure 5 is a schematic view of insertion of the cold-store material into the respective receptacle through the apertures.
Figure 6 shows a detail of the internal structure that forms the receptacles arranged in the form of a quincunx, portraying a brick-wall pattern.
Figure 7 is a schematic perspective view of a possible embodiment of the pack of cold-store material.
Figure 8 is another schematic perspective view of the same embodiment of the pack of cold-store material as in Figure 7.
Figure 9 is a schematic plan view of the same embodiment of the pack of cold-store material as in Figure 7.
Figure 10 is a schematic side elevation view of the same embodiment of the pack of cold-store material as in Figure 7.

In the figures, the same or equivalent elements have been designated using the same numerals.

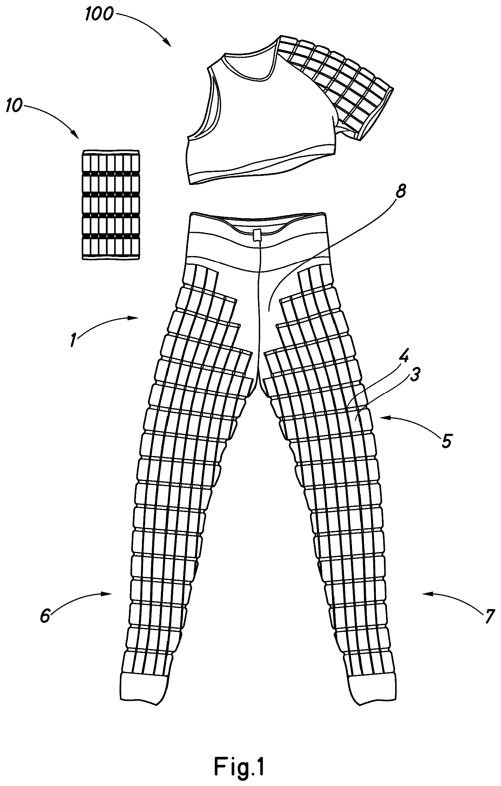

Figure 1 shows a set of various example embodiments of garments 1, 10 and 100 for cryotherapy according to the present invention. As shown, the garment 1 takes the form of long leggings, i.e. leggings that reach approximately to the user's ankles, while the garment 10 takes the form of a sleeve, and the garment 100 takes the form of a shirt. Alternatively, the leggings may be short, i.e. reaching to above the knee, or pedal pushers, i.e. reaching approximately the midpoint of the calves. The trouser legs in the example, including the receptacles, can be made using a circular machine.

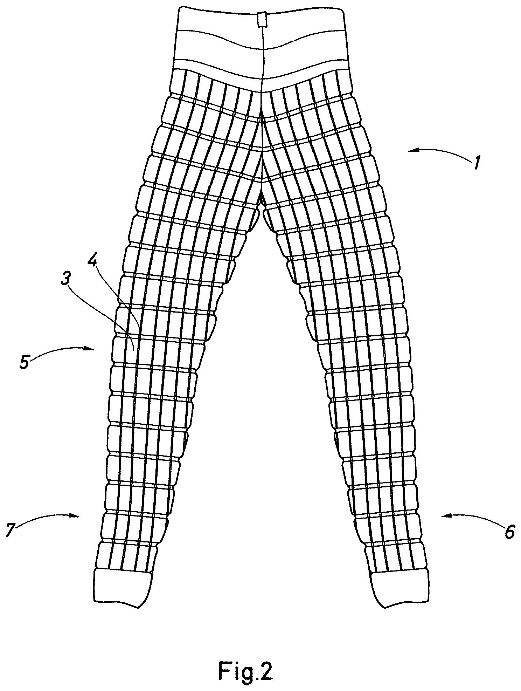

Figure 2 shows the reverse of the garment 1 in the same embodiment shown in Figure 1. Said garment 1 has two legs 6 and 7, each of which has a respective plurality of receptacles 3 for receiving a cold-store material 2 (not shown).

As shown, the different receptacles 3 are positioned next to one another, forming a set of rows 5 spaced apart substantially equidistantly from one another, although in other embodiments said gaps may vary depending on the area of the body where the receptacles are positioned and the desired effect, among other aspects. The gaps between rows 5 of receptacles may also be determined for aesthetic reasons. Similarly, the distance between the receptacles within the rows can also vary, either depending on the area of the body where the receptacles are positioned, the desired effect, or aesthetic reasons.

As shown, the rows 5 of receptacles in this embodiment are arranged essentially perpendicularly to the longitudinal axis of the respective trouser leg 6 and 7, i.e. substantially perpendicular to the longitudinal axis of the leg of the user of the garment 1. The receptacles that form said rows 5 are arranged essentially parallel to the longitudinal axis of the respective trouser leg thereof. In other words, the pockets are vertical and the rows are horizontal, when the garment is being worn and the user is in a standing position. As a result, the receptacles are arranged in a grid or matrix pattern. Similarly, in embodiments of garments in which the garment covers the arms and/or the torso of the user of the garment, as is the case with the shirt 100 or the sleeve 10, the receptacles and the respective cold-store material thereof can also be arranged in the same matrix pattern, with rows 5 that are substantially perpendicular to the longitudinal axis of the arms and/or the torso formed by a set of adjacent receptacles 3 arranged parallel to said axis.

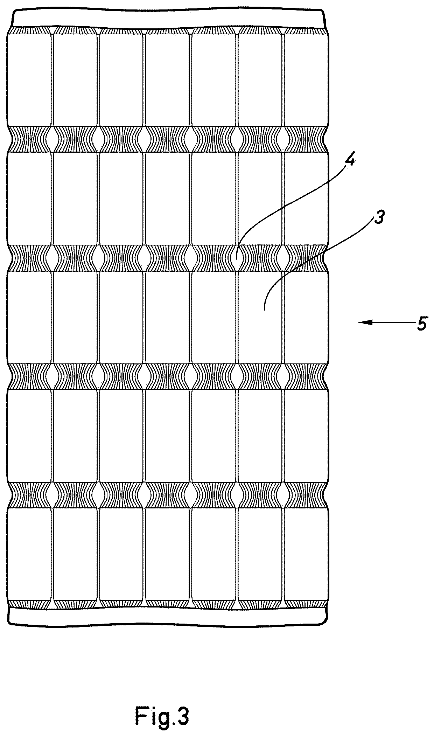

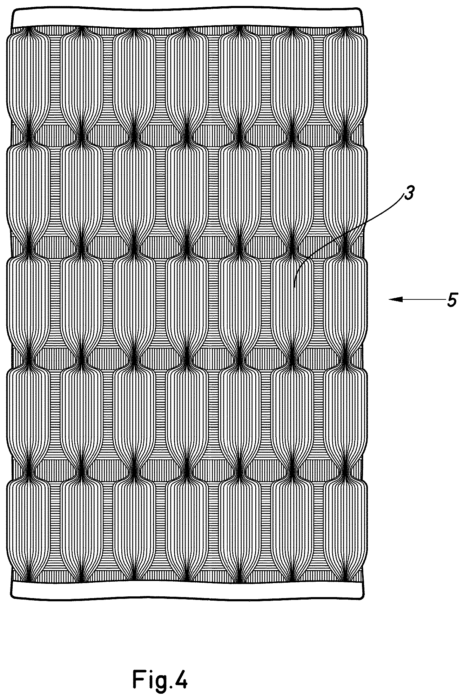

Figure 3 shows the receptacles 3 of the garment 10 and the formation thereof in greater detail, and Figure 4 shows the inside of the garment 10 of the same embodiment.

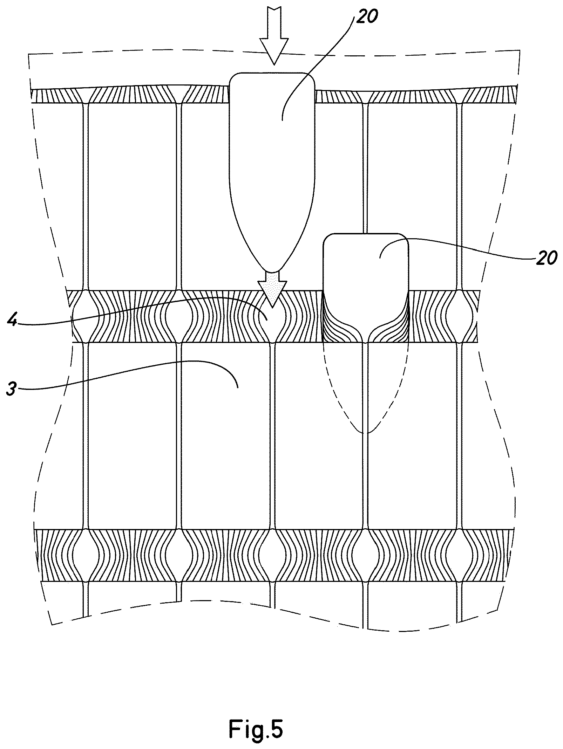

Figure 5 shows a detail of how the pack 20 of the cold-store material 2 is inserted into the receptacles 3. The receptacles 3 are formed from folds of elastic material through a structure, of at least two layers, for example woven using a circular machine. Said folds are sealed on all sides except one end, where there is an opening 4. Said opening 4 is usually positioned at the upper end to help hold the pack inside the receptacle when putting the garment on the body, since friction may push the cold-store material towards the bottom of the receptacle and also helps to counteract the effect of gravity once in position. Notwithstanding the foregoing, said opening 4 may be positioned at the bottom or even on a lateral side if the orientation or location of the receptacle in the garment is such that the positioning of the pack of cold-store material is more ergonomic for the user.

The receptacles 3 are folds forming tubular pockets that have an opening 4 with a perimeter smaller than the transverse perimeter of the pack 20 of cold-store material 2. The transverse perimeter of the receptacles 3 is also smaller than the transverse perimeter of said pack 20. Since the fabric used to make the entire garment, including receptacles 3 and openings 4, is made of elastic material, the opening 4 can be widened to enable passage of the pack 20, and the receptacle 3 can be fitted to and hold said pack 20 by constriction. This figure shows how a differently coloured layer of the structure is centred in front of the pack 20, thereby forming a line that resembles a seam.

It should be noted that if the elasticity of the receptacles 3 so permits, another type of pack of cold-store material can be inserted, or solid cold-store material such as ice cubes can be inserted directly.

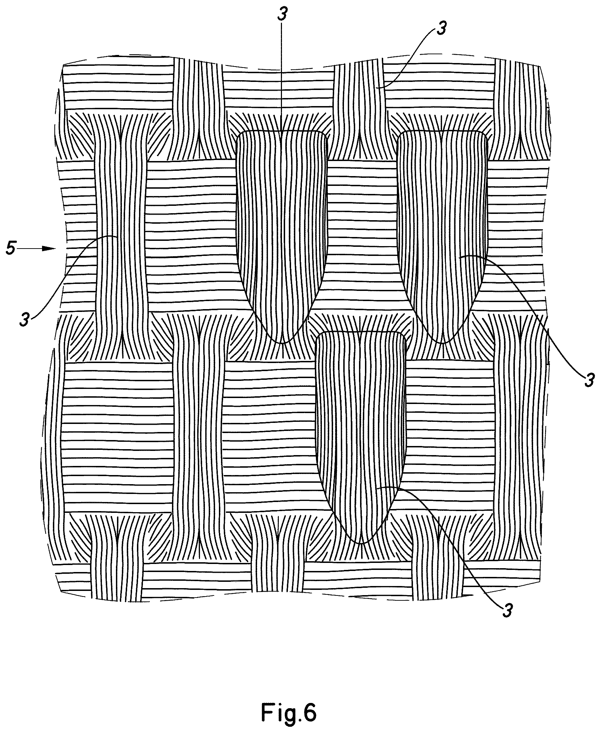

Figure 6 shows a detail of the receptacles 3 and the formation thereof according to a different embodiment, in which said receptacles 3 are also arranged in rows 5 perpendicular to the longitudinal axis of the limb or the body to be covered, said rows being offset from one another such that the resulting overall arrangement between the receptacles 3 is a quincunx arrangement. This arrangement facilitates the insertion or removal of the packs 20 of cold-store material 2 with respect to the receptacles 3, since there is less interference between said inserted material 2 and the material to be inserted and removed, since the openings 4 are between the upper and lower receptacles 3.

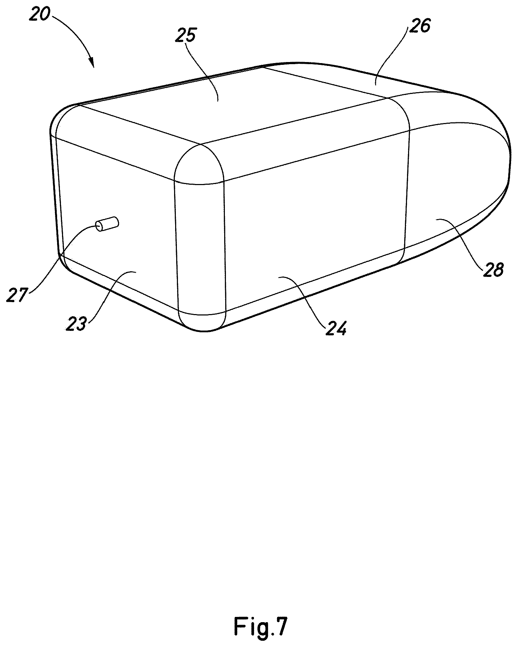

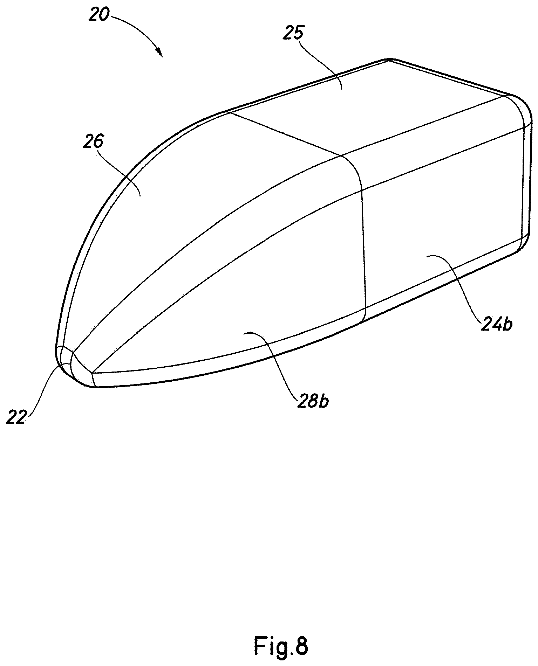

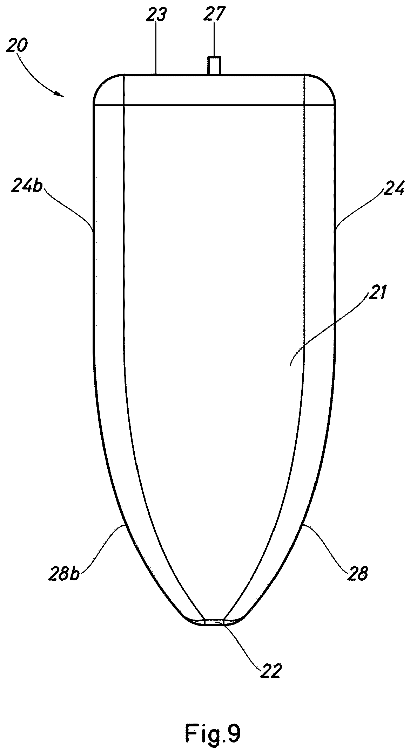

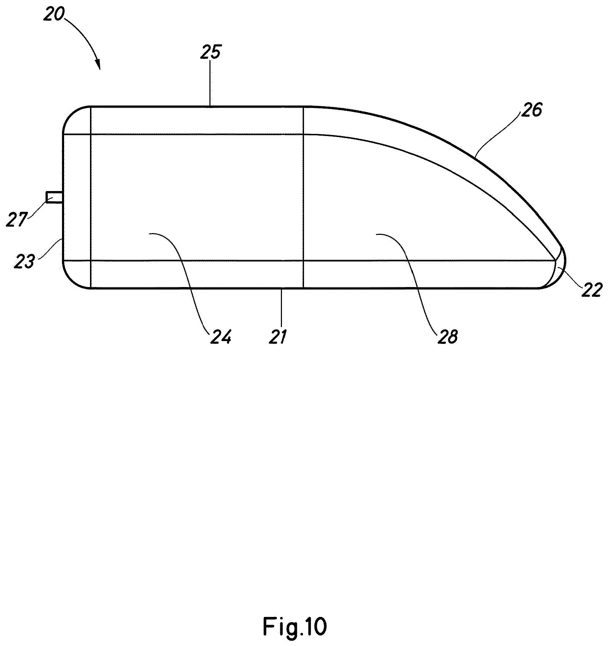

Figures 7-10 show the pack 20 of cold-store material 2 in detail. The pack 20 is made of semi-rigid plastic and the cold-store material 2 is a fluid (not shown) with a low melting point and high thermal inertia, said fluid preferably having low viscosity, for example distilled water or alcohol water, although said fluid may also be a gel. Said pack 20 containing the fluid has at least one flat face 21, said at least one flat face being the face of the pack 20 with the largest surface area that faces the body when the garment is in use, having been inserted in the receptacle, on account of which said face can be considered to be the base or bottom face of the pack 20. The shape of said pack 20 is preferably similar to the first unit of a bullet train or high-speed train, comprising the aforementioned flat face 21 with the largest surface area, a rounded three-dimensional point 22, a rear flat face 23 at the other end, two rear side flat faces 24 and 24b, a rear top flat face 25, two front side curved surfaces 28 and 28b that join respectively the side faces 24 and 24b to the rounded three-dimensional point 22, and a front upper curved surface 26 that joins the rear upper face 25 to the point 22. In this embodiment, there is a projection 27 on the rear flat face 23 resulting from the sealing of the pack 20 after the fluid has been inserted. In other embodiments, this projection 27 may be absent, for example if the pack 20 is sealed using a method that does not result in a projection of material, or if the projection is trimmed after sealing. In other embodiments, said projection 27 may be used to help remove the material 2 from the receptacles 3, adapting the projections to be easy to grasp and pull, or joining the projection to a thread or set of threads that can be pulled. Similarly, in other embodiments, the packs can have a number of different surfaces, depending on the radii of curvature and the tangents between surfaces.

In another embodiment that is not shown, each of the legs 6 and 7 of the garment 1 can have a zone with no receptacles 3 or cold-store material 2. Said zones can coincide approximately with the location of the joints of the user of the garments. For the garment 1, the zones could be the knees and/or ankles, and for the garment 10 or 100, said zone with no cold-store material could preferably be the zone about the user's elbow.

In the embodiment showing a set of garments, the garment 1 has a zone 8 with no cold-store material that coincides with the genital area of the user of the garment 1. Among other objectives, the absence of cold-store material is intended to increase usage comfort of the garment 1, since said zone is particularly sensitive to the cold.

In the aforementioned embodiments of the invention, the fabric of the garments, in addition to embodying the different garments 1, 10 and 100 and holding the cold-store material 2, is also used to prevent direct contact between said cold-store material 2 and the skin of the user of said garment, which could cause wounds and/or ice burns to said user. Although the fabric of the garment 10 in the example embodiment shown in Figure 4 only has one layer, the fabric can have more than one layer in other embodiments.

Although the figures show and describe an example embodiment of garments according to the present invention in which said garments are leggings, sleeves or short-sleeve shirts, the invention should also be understood to apply equally to embodiments in which the garment is a long sleeve shirt, a calf sleeve, etc.

Moreover, although the figures show and describe a design of the packs for the cold-store material, other designs may be used to optimise the cited handling parameters for the cold-store material and the cryogenic effect that said material can have for the user.

An example object of said optimisation could be a pack in which the flat face of largest surface area is formed by five straight sides forming an elongate pentagon that is symmetrical about the longitudinal axis thereof. As in the embodiment shown, said pentagon would have three sides with two right angles, but the point would be formed by two straight sides forming an obtuse angle with the lateral sides. This would slightly increase the surface area of the flat face used for treatment without significantly adversely affecting handling of the material in the receptacles.

Another object of said optimisation could be a design in which the flat face of largest surface area is an elongate hexagon that is pointed at both ends thereof. This would facilitate both the insertion and extraction of the material to and from the receptacles. Combining this design with the embodiment in which the receptacles are arranged in a quincunx pattern would arrange the receptacles in the form of a swarm of bees, which would further reduce the space between the receptacles, thereby further optimizing the surface area used for treatment.

Although the invention has been described and illustrated with reference to a number of representative examples, it will be appreciated that these exemplary embodiments are in no way limiting on the present invention and therefore any of the variants included, directly or as equivalents, within the content of the attached claims must be considered to be included within the scope of the present invention.

## Claims

1. Cryotherapy garment including various receptacles designed to receive at least one pack of cold-store material in which each of said receptacles is formed by a closed fabric fold in the garment, each of said receptacles having an opening for insertion of a pack of cold-store material, each receptacle receiving a respective pack, **characterized in that** said packs are made of a semi-rigid material, and have at least one flat face, said flat face being the face with the largest surface area of the corresponding pack, said flat face of the pack being in contact, following insertion thereof into an opening of a receptacle, with the surface of the garment designed to come into contact in turn with the user of the garment, said flat face of the packs being delimited by three flat faces two of them being at opposite sides of the flat face, forming two right angles between said three flat faces and two curved surfaces each being aligned with one of the flat faces that are placed opposite to each other, that together with a top curved surface form a pointed end to facilitate insertion of said packs into the respective receptacles thereof, the top curved surface also joining the three flat faces with a top flat face

2. Garment according to the preceding claim, **characterized in that** the flat face of the packs is delimited by six sides forming a hexagon.

3. Garment according to any of the preceding claims, **characterized in that** said fabric of the garment includes an elastic material.

4. Garment according to the preceding claims, **characterized in that** each receptacle has a hole of smaller dimensions than the dimensions of the pack contained therein.

5. Garment according to the preceding claims, **characterized in that** the receptacles are tubular pockets.

6. Garment according to the preceding claims, **characterized in that** said folds are closed longitudinally using weaving techniques along one or two closing lines.

7. Garment according to the preceding claims, **characterized in that** the receptacles are arranged next to one another to form horizontal rows.

8. Garment according to claim 7 **characterized in that** at least one of said horizontal rows extends about the entire perimeter of the body or limb to be covered.

9. Garment according to claim 7 or 8, **characterized in that** at least two horizontal rows are arranged such that the receptacles thereof are also vertically aligned.

10. Garment according to claim 7 or 8, **characterized in that** the receptacles are arranged in offset rows, following an overall quincunx pattern.

11. Garment according to any of the preceding claims, **characterized in that** the longitudinal axis of said receptacles is substantially parallel to the longitudinal axis of the respective limb thereof.

12. Garment according to any of the preceding claims, **characterized in that** said cold-store material is distilled water.

## Patentansprüche

1. Kryotherapie-Kleidungsstück, aufweisend verschiedene Aufnahmen, die ausgestaltet sind, mindestens einen Pack mit Kältespeichermaterial aufzunehmen, wobei jede dieser Aufnahmen durch eine geschlossene Stofffalte im Kleidungsstück gebildet wird, wobei jede dieser Aufnahmen eine Öffnung zum Einführen eines Packs mit Kältespeichermaterial aufweist und jede Aufnahme ein jeweiliges Pack aufnimmt, **dadurch gekennzeichnet, dass** die Packs aus einem halbstarren Material bestehen und mindestens eine flache Fläche aufweisen, wobei die flache Fläche die Fläche mit der größten Oberfläche des entsprechenden Packs ist, wobei die flache Fläche des Packs nach dem Einführen in die Öffnung einer Aufnahme mit der Oberfläche des Kleidungsstücks in Kontakt steht, wobei die Oberfläche des Kleidungsstücks ausgestaltet ist, wiederum mit dem Benutzer des Kleidungsstücks in Kontakt zu kommen, wobei die flache Fläche der Packs durch drei flache Flächen begrenzt ist, von denen sich zwei an gegenüberliegenden Seiten der flachen Fläche befinden und zwei rechte Winkel zwischen den drei flachen Flächen bilden, sowie durch zwei gekrümmte Flächen, die jeweils mit einer der einander gegenüberliegenden flachen Flächen ausgerichtet sind und zusammen mit einer oberen gekrümmten Fläche ein spitzes Ende bilden, um das Einführen der Packs in die jeweiligen Aufnahmen zu erleichtern, wobei die obere gekrümmte Fläche die drei flachen Flächen außerdem mit einer oberen flachen Fläche verbindet.

2. Kleidungsstück nach dem vorstehenden Anspruch, **dadurch gekennzeichnet, dass** die flache Fläche der Packs durch sechs Seiten begrenzt ist, die ein Sechseck bilden.

3. Kleidungsstück nach einem der vorstehenden Ansprüche, **dadurch gekennzeichnet, dass** der Stoff des Kleidungsstücks ein elastisches Material aufweist.

4. Kleidungsstück nach den vorstehenden Ansprüchen, **dadurch gekennzeichnet, dass** jede Aufnahme ein Loch aufweist, dessen Abmessungen kleiner sind als die Abmessungen des darin enthaltenen Packs.

5. Kleidungsstück nach den vorstehenden Ansprüchen, **dadurch gekennzeichnet, dass** die Aufnahmen röhrenförmige Taschen sind.

6. Kleidungsstück nach den vorstehenden Ansprüchen, **dadurch gekennzeichnet, dass** die Falten in Längsrichtung unter Verwendung von Webtechniken entlang von einer oder zwei Verschlusslinien geschlossen sind.

7. Kleidungsstück nach den vorstehenden Ansprüchen, **dadurch gekennzeichnet, dass** die Aufnahmen nebeneinander angeordnet sind, um horizontale Reihen zu bilden.

8. Kleidungsstück nach Anspruch 7, **dadurch gekennzeichnet, dass** sich mindestens eine der horizontalen Reihen um den gesamten Umfang des zu bedeckenden Körpers oder Glieds erstreckt.

9. Kleidungsstück nach Anspruch 7 oder 8, **dadurch gekennzeichnet, dass** mindestens zwei horizontale Reihen so angeordnet sind, dass ihre Aufnahmen auch vertikal ausgerichtet sind.

10. Kleidungsstück nach Anspruch 7 oder 8, **dadurch gekennzeichnet, dass** die Aufnahmen in versetzten Reihen angeordnet sind, die einem Quincunx-Muster folgen.

11. Kleidungsstück nach einem der vorstehenden Ansprüche, **dadurch gekennzeichnet, dass** die Aufnahmen eine Längsachse aufweisen, die im Wesentlichen parallel zur Längsachse des jeweiligen Glieds verläuft.

12. Kleidungsstück nach einem der vorstehenden Ansprüche, **dadurch gekennzeichnet, dass** das Kältespeichermaterial destilliertes Wasser ist.

## Revendications

1. Vêtement de cryothérapie comprenant divers réceptacles destinés à recevoir au moins un sachet de matériau réfrigérant, chacun desdits réceptacles étant formé par un pli fermé du tissu du vêtement, chacun desdits réceptacles ayant une ouverture pour l'insertion d'un sachet de matériau réfrigérant, chaque réceptacle recevant un sachet respectif, **caractérisé en ce que** lesdits sachets sont réalisés en un matériau semi-rigide et ont au moins une face plane, ladite face plane étant la face présentant la plus grande surface du sachet correspondant, ladite face plane du sachet étant en contact, après son insertion dans une ouverture d'un réceptacle, avec la surface du vêtement conçue pour entrer en contact à son tour avec l'utilisateur du vêtement, ladite face plane des sachets étant délimitée par trois faces planes, deux d'entre elles se trouvant sur les côtés opposés de la face plane, formant deux angles droits entre lesdites trois faces planes et deux surfaces courbes, chacune étant alignée avec l'une des faces planes qui sont placées à l'opposé l'une de l'autre, qui, avec une surface courbe supérieure, forment une extrémité pointue pour faciliter l'insertion desdits sachets dans leurs réceptacles respectifs, la surface courbe supérieure reliant également les trois faces planes à une face plane supérieure.

2. Vêtement selon la revendication précédente, **caractérisé en ce que** la face plane des sachets est délimitée par six côtés formant un hexagone.

3. Vêtement selon l'une quelconque des revendications précédentes, **caractérisé en ce que** ledit tissu du vêtement comprend un matériau élastique.

4. Vêtement selon les revendications précédentes, **caractérisé en ce que** chaque réceptacle a un trou de dimensions inférieures aux dimensions du sachet qu'il contient.

5. Vêtement selon les revendications précédentes, **caractérisé en ce que** les réceptacles sont des poches tubulaires.

6. Vêtement selon les revendications précédentes, **caractérisé en ce que** lesdits plis sont fermés longitudinalement à l'aide de techniques de tissage le long d'une ou deux lignes de fermeture.

7. Vêtement selon les revendications précédentes, **caractérisé en ce que** les réceptacles sont disposés les uns à côté des autres pour former des rangées horizontales.

8. Vêtement selon la revendication 7, **caractérisé en ce qu'**au moins une desdites rangées horizontales s'étend sur tout le périmètre du corps ou du membre à couvrir.

9. Vêtement selon la revendication 7 ou 8, **caractérisé en ce qu'**au moins deux rangées horizontales sont disposées de telle sorte que leurs réceptacles soient également alignés verticalement.

10. Vêtement selon la revendication 7 ou 8, **caractérisé en ce que** les réceptacles sont disposés en rangées décalées, suivant un motif global en quinconce.

11. Vêtement selon l'une quelconque des revendications précédentes, **caractérisé en ce que** l'axe longitudinal desdits réceptacles est sensiblement parallèle à l'axe longitudinal du membre respectif.

12. Vêtement selon l'une quelconque des revendications précédentes, **caractérisé en ce que** ledit matériau réfrigérant est de l'eau distillée.
